# EUROPÄISCHE PATENTANMELDUNG

(11) **EP 2 243 464 A2**
(43) Veröffentlichungstag der Anmeldung: **27.10.2010**
(21) Anmeldenummer: 10155041.6
(22) Anmeldetag: 01.03.2010
(51) Int. Cl.: A61K 8/35, A61K 8/37, A61K 8/44, A61K 8/49, A61K 8/64, A61K 8/66, A61K 8/97, A61K 8/99, A61Q 19/08

(54) **Hautbehandlungsmittel gegen Hautalterung I**

(30) Priorität: 20.04.2009 DE 102009017612
(71) Anmelder: Henkel AG & Co. KGaA, 40589 Düsseldorf (DE)
(72) Erfinder: Janßen, Frank, 41470, Neuss (DE); Dickhof, Susanne, 41748, Viersen (DE); Emond, Eliane, 40470, Düsseldorf (FR)

(57) **Zusammenfassung**

Kosmetische oder dermatologische topische Zusammensetzungen, enthaltend in einem geeigneten kosmetischen oder dermatologischen Träger - jeweils bezogen auf das Gewicht der gesamten Zusammensetzung -0,001 bis 10 Gew.-% mindestens eines Extraktes aus Laminaria Digitata und 0,001 bis 10 Gew.-% mindestens eines Wirkstoffes aus der Gruppe
- der 6,7-disubstituierten 2,2-Dialkylchromane oder -chromene der allgemeinen Formeln (I) order (II), 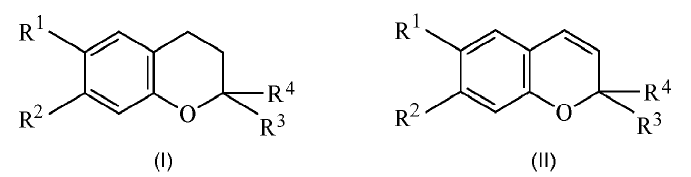 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6 und/oder
- Ellagsäure und/oder Ellagate und/oder
- der Xanthophylle, insbesondere Astaxanthin, und/oder
- der Superoxiddismutasen und/oder
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten und/oder
- Monomeren, Oligomeren und Polymeren von Aminosäuren, N-C₂-C₂₄-Acylaminosäuren, den Estern und/oder den physiologisch verträglichen Metallsalzen dieser Substanzen,
eignen sich hervorragend zum Schutz vor und zur Behandlung von Hautalterungserscheinungen.

## Beschreibung

Die Erfindung betrifft topische kosmetische oder dermatologische Zusammensetzungen zur Hautbehandlung, die der Hautalterung und deren Anzeichen entgegenwirken.

Mittel und Behandlungsmethoden zur Behandlung reifer oder intrinsisch oder extrinsisch gealterter oder lichtgeschädigter Haut, insbesondere zur Antifaltenbehandlung, sind eine bedeutende kosmetische Herausforderung. Der Alterungsprozess ist gekennzeichnet durch einen fortschreitenden Übergang der Hautzellen aus einem proliferativen Status in einen ruhenden, seneszenten Status. Dieser Übergang entspricht der Alterung auf zellulärer Ebene, auf den sich ein Großteil der makroskopischen Alterungseffekte zurückführen lässt. Die Zellzahl im Gewebe verringert sich und kann mangels proliferierender Zellen nicht mehr aufgefüllt werden. Dadurch können keine Reparaturen des umliegenden Gewebes durchgeführt werden, Defekte reichern sich an, wodurch die Haut atrophisch wird und erschlafft. Verstärkt wird dieser Effekt unter anderem auch durch UV-Licht, das die Haut ebenfalls belastet und deren Alterung und Erschlaffung beschleunigt.

Der vorliegenden Erfindung lag die Aufgabe zugrunde, Zubereitungen zur Behandlung und deutlichen Reduzierung der Erscheinungsformen der Hautalterung bereitzustellen, die bislang marktübliche Produkte in der Leistung übertreffen. Es war eine weitere Aufgabe der vorliegenden Erfindung, eine neue, stabile und kosmetisch wirksame Hautpflegezubereitung zur Prophylaxe und Behandlung von Hautalterungserscheinungen, insbesondere Fältchen und Falten zu entwickeln. Insbesondere sollte durch die Hautpflegezubereitung die Hautfeuchtigkeit erhöht werden.

Es wurde nun festgestellt, dass die bekannten Zusammensetzungen in ihren Eigenschaften verbessert werden können, wenn bestimmte an sich bekannte Inhaltsstoffe in diesen Mitteln kombiniert werden.

Gegenstand der vorliegenden Erfindung sind in einer ersten Ausführungsform kosmetische oder dermatologische topische Zusammensetzungen, enthaltend in einem geeigneten kosmetischen oder dermatologischen Träger - jeweils bezogen auf das Gewicht der gesamten Zusammensetzung -
a) 0,001 bis 10 Gew.-% mindestens eines Extraktes aus Laminaria Digitata und
b) 0,001 bis 10 Gew.-% mindestens eines Wirkstoffes aus der Gruppe
   - der 6,7-disubstituierten 2,2-Dialkylchromane oder -chromene der allgemeinen Formeln (I) oder (II), 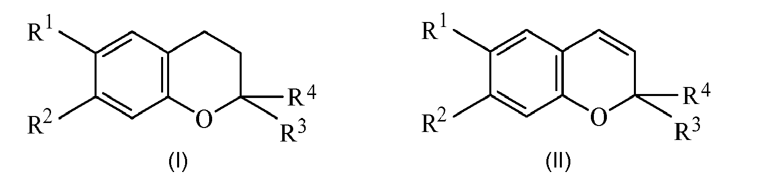 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6 und/oder
   - Ellagsäure und/oder Ellagate und/oder
   - der Xanthophylle, insbesondere Astaxanthin, und/oder
   - der Superoxiddismutasen und/oder
   - der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
   - Milchsäurebakterien-basierten Fermentationen und/oder
   - Ethylhexenoat und seinen Derivaten und/oder
   - Methylbutyrat und seinen Derivaten und/oder
   - Ethyldecadienoat und seinen Derivaten und/oder
   - Monomeren, Oligomeren und Polymeren von Aminosäuren, N-C₂-C₂₄-Acylaminosäuren,
      den Estern und/oder den physiologisch verträglichen Metallsalzen dieser Substanzen.
      Die erfindungsgemäßen Zusammensetzungen enthalten mindestens einen Extrakt aus Laminaria Digitata. Die Laminariales sind eine Ordnung der Braunalgen, deren Mitglieder unter Wasser im klaren, flachen Meer Wälder bilden. Mehrere Gattungen werden als Kelp bezeichnet, der Rest mit dem allgemeinen Wort "Tang". Die Art Laminaria Digitata wird auch als Fingertang, Blatt- oder Riementang bezeichnet. Die Extraktion von Laminaria Digitata liefert Laminarin als Hauptbestandteil. Laminarin ist ein lineares Glucan mit beta-1,3-glycosidischen und einzelnen beta-1,6-glycosidischen Bindungen. Erfindungsgemäß bevorzugte Zusammensetzungen enthalten Laminarin. Erfindungsgemäß besonders bevorzugte Zusammensetzungen sind **dadurch gekennzeichnet, dass** sie 0,00005 bis 0,5 Gew.-%, vorzugsweise 0,0005 bis 0,25 Gew.-% und insbesondere 0,005 bis 0,1 Gew.-% Laminarin der Formel (III) enthalten 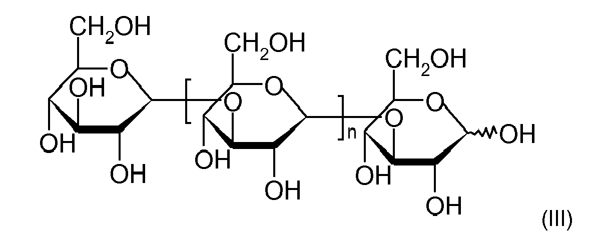 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind.
      Als zweiten wesentlichen Inhaltsstoff enthalten die erfindungsgemäßen Mittel mindestens einen Stoff aus der Gruppe
   - der 6,7-disubstituierten 2,2-Dialkylchromane oder -chromene der allgemeinen Formeln (I)
      oder (II), 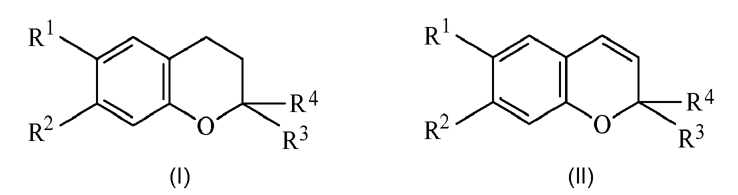 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6 und/oder
   - Ellagsäure und/oder Ellagate und/oder
   - der Xanthophylle, insbesondere Astaxanthin, und/oder
   - der Superoxiddismutasen und/oder
   - der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
   - Milchsäurebakterien-basierten Fermentationen und/oder
   - Ethylhexenoat und seinen Derivaten und/oder
   - Methylbutyrat und seinen Derivaten und/oder
   - Ethyldecadienoat und seinen Derivaten und/oder
   - Monomeren, Oligomeren und Polymeren von Aminosäuren, N-C₂-C₂₄-Acylaminosäuren,
      den Estern und/oder den physiologisch verträglichen Metallsalzen dieser Substanzen.
      Diese Stoffe werden nachstehend beschrieben.
      Als erstes besonders geeignetes Antioxidans können die erfindungsgemäßen Mittel 6,7-disubstituierte 2,2-Dialkylchromane oder -chromene der allgemeinen Formeln (I) oder (II), 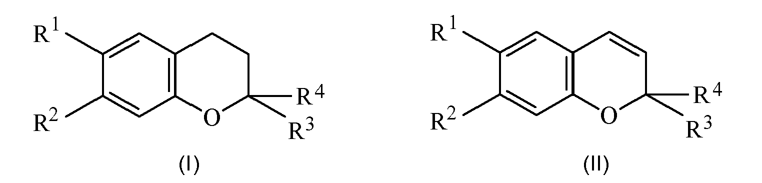 enthalten, wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen. Die Substituenten R¹ und R² sind unabhängig voneinander ausgewählt aus einer OH-Gruppe, einer Methoxy-Gruppe und einer CF₃CH₂O-Gruppe. In einer bevorzugten Ausführungsform sind R¹ und R² unabhängig voneinander ausgewählt aus einer OH-Gruppe und einer Methoxy-Gruppe. In einer besonders bevorzugten Ausführungsform stellen R¹ eine OH-Gruppe und R² eine Methoxy-Gruppe dar.

Die Substituenten R³ und R⁴ stellen unabhängig voneinander eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind R³ und R⁴ unabhängig voneinander ausgewählt aus einer Methyl-, Ethyl-, n-Propyl-, Isopropyl- und einer n-Butyl-Gruppe. Besonders bevorzugt stellen R³ und R⁴ unabhängig voneinander eine Methyl- oder Ethylgruppe dar. Außerordentlich bevorzugt ist, dass R³ und R⁴ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (I) oder (II) werden vorzugsweise in einer Gesamtmenge von 0,001 - 5 Gew.-%, bevorzugt 0,005 - 2 Gew.-% und besonders bevorzugt 0,01 - 1 Gew.-%, jeweils bezogen auf die gesamte Zusammensetzung, eingesetzt.

Zusammenfassend sind erfindungsgemäße Zusammensetzungen bevorzugt, die - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (I) oder (II) wie vorstehend definiert, enthalten, wobei bevorzugte Mittel - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-%, insbesondere 0,005 bis 2 Gew.-%, besonders bevorzugt 0,01 - 1 Gew.-% und insbesondere 0,05 - 0,1 Gew.-% Lipochroman-6 enthalten.

Als ein weiteres besonders geeignetes Antioxidans können die erfindungsgemäßen Mittel mindestens einen Stoff aus der Gruppe der Ellagsäure und/oder Ellagate enthalten. Ellagsäure (2,3,7,8-Tetrahydroxy[1]benzopyrano-[5,4,3-*cde*][1]benzopyran-5,10-dion 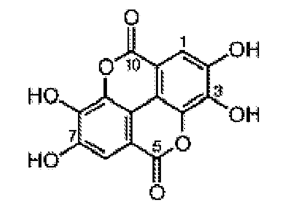 ist frei und in Form von Glycosiden und/oder Methylethern im Pflanzenreich weit verbreitet, besonders in Galläpfeln, Blattgallen, auch in den Blättern von *Eucalyptus*-Arten, in Eichen und Euphorbien.

Bevorzugte erfindungsgemäße Zusammensetzungen sind **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Ellagsäure (2,3,7,8-Tetrahydroxy[1]benzopyrano-[5,4,3-cde][1]benzopyran-5,10-dion) und/oder Ellagate enthalten.

Als ein weiteres besonders geeignetes Antioxidans können die erfindungsgemäßen Mittel mindestens einen Stoff aus der Gruppe der Xanthophylle, insbesondere Astaxanthin, enthalten. Xanthophylle ist der Gruppenname für Hydroxy-, Epoxy- und Oxo-Derivate von Carotinoiden. Beispiele für erfindungsgemäß bevorzugte Xanthophylle sind z. B. Canthaxanthin, Citranaxanthin, Flavoxanthin, Rhodoxanthin, Rubixanthin, Zeaxanthin, Lutein, Cryptoxanthin, Violaxanthin und insbesondere Astaxanthin. Die natürlich vorkommenden Xanthophylle sind meist *all*-*trans*-Verbindungen. Erfindungsgemäß bevorzugte Zusammensetzungen enthalten - bezogen auf ihr Gewicht - 0,001 bis 10 Gew.-%, vorzugsweise 0,005 - 7,5 Gew.-%, weiter bevorzugt 0,01 - 5 Gew.-%, insbesondere 0,02 - 1 Gew.-%, besonders bevorzugt 0,05 - 0,1 Gew.-%, Xanthophylle, vorzugsweise Canthaxanthin, Citranaxanthin, Flavoxanthin, Rhodoxanthin, Rubixanthin, Zeaxanthin und ganz besonders bevorzugt Astaxanthin.

Als ein weiteres besonders geeignetes Antioxidans können die erfindungsgemäßen Mittel mindestens einen Stoff aus der Gruppe der Superoxiddismutasen enthalten. Erfindungsgemäß bevorzugte Zusammensetzungen enthalten- bezogen auf ihr Gewicht - 0,001 bis 10 Gew.-%, vorzugsweise 0,005 - 7,5 Gew.-%, weiter bevorzugt 0,01 - 5 Gew.-%, insbesondere 0,02 - 2 Gew.-%, weiter bevorzugt 0,05 - 0,1 Gew.-%, Superoxiddismutase(n).

Anstelle der vorstehend genannten Antioxidantien oder in Ergänzung zu ihnen können die erfindungsgemäßen Mittel auch Wirkstoffe enthalten, die die enzymatische Aktivität oder die Expression der L-Isoform der Lysyloxidase (LOXL) stimulieren.

Mit dem Begriff "LOXL" oder "hLOXL" ist die L-Isoform des humanen Proteins Lysyloxidase LOXL gemeint. Mit dem Begriff "LOX" oder "hLOX" ist die Anfangs-Isoform ("Pisoform initiale" des humanen Proteins Lysyloxidase LOX gemeint. Mit "Stimulieren der Expression der Isoform der Lysyloxidase LOXL" ist die Stimulierung der Expression des Gens, das für LOXL kodiert, oder von dessen Promotor gemeint, und insbesondere ist die Stimulierung der Synthese der messenger RNA, die für LOXL kodiert, und ebenfalls die Stimulierung der Synthese von LOXL, das aus dieser messenger RNA resultiert, gemeint. Mit "Stimulieren der Expression von Elastin der Isoform der Lysyloxidase LOXL" ist die Stimulierung der Expression des Gens, das für das Protein Elastin kodiert, oder von dessen Promotor gemeint, und insbesondere ist die Stimulierung der Synthese der messenger RNA, die für das Protein Elastin kodiert, und ebenfalls die Stimulierung der Synthese des Proteins Elastin oder seines Vorläufers, Tropoelastin, das aus dieser messenger RNA resultiert, gemeint.

Wirkstoffe sind dann als wirksam anzusehen, wenn sie eine Aktivierung oder einen 1,5-fachen Anstieg der Expression und/oder der Aktivität von LOXL in einem Modell erzielen, das mindestens einen Zelltyp umfasst, der eine Expression und/oder Aktivität von LOXL über den Kontakt mit diesen Wirkstoffen aufweist, in Bezug auf den Expressionslevel und/oder Aktivitätslevel von LOXL in einem Kontrollmodell (ohne Kontakt mit den Wirkstoffen).

Vorteilhafterweise ist die Expression von LOXL entweder die Expression einer Nukleotidsequenz, die für LOXL kodiert, oder die Expression einer Aminosäuresequenz, die eine Teilsequenz des Proteins LOXL darstellt.

Vorteilhafterweise wird die Wirksubstanz ausgewählt aus der Gruppe
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten.

Anstelle der vorstehend genannten Antioxidantien und/oder der Wirkstoffe, die die enzymatische Aktivität oder die Expression der L-Isoform der Lysyloxidase (LOXL) stimulieren und/ oder in Ergänzung zu ihnen können die erfindungsgemäßen Mittel auch Monomere, Oligomere und Polymere von Aminosäuren, N-C₂-C₂₄-Acylaminosäuren, den Estern und/oder den physiologisch verträglichen Metallsalzen dieser Substanzen enthalten.

Bevorzugte erfindungsgemäße Zusammensetzungen sind **dadurch gekennzeichnet, dass** die Monomeren von Aminosäuren, N-C₂-C₂₄-Acylaminosäuren und/oder den physiologisch verträglichen Metallsalzen dieser Substanzen ausgewählt sind aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von Seppic erhältlich ist.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere können als Wirkstoffe gegen die Hautalterung verwendet werden.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und lle geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gln und den Aminosäuren, die Gln substituieren können, Xcc ausgewählt ist aus Pro oder Arg und den Aminosäuren, die Pro und Arg substituieren können.

Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).

Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.

Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.

Als Rigin-Analoga sind erfindungsgemäß Gly-Gln-Arg-Pro und Val-Val-Arg-Pro bevorzugt.

ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1 Ein weiteres bevorzugtes Aminosäureoligomer ist das Peptidderivat L-Glutamylaminoethyl-indol (Glistin von Exsymol).

Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.

Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet. Proteinhydrolysate können naturgemäß auch monomere Aminosäuren und Oligopeptide enthalten; ihre Zusammensetzung ist normalerweise nicht definiert.

Ebenfalls bevorzugt ist der Einsatz von Acylderivaten der Proteinhydrolysate, z. B. in Form ihrer Fettsäure-Kondensationsprodukte.

Zusammenfassend sind erfindungsgemäß bevorzugte Zusammensetzungen dadurch gekennzeichnet, dass die Oligomeren von Aminosäuren, N-C₂-C₂₄-Acylaminosäuren und/oder den physiologisch verträglichen Metallsalzen dieser Substanzen ausgewählt sind aus Di-, Tri-, Tetra-, Penta-, Hexa- oder Pentadecapeptiden, die N-acyliert und/oder verestert sein können.

Ganz besonders bevorzugte erfindungsgemäße Zusammensetzungen sind dadurch gekennzeichnet, dass das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg.

Ebenfalls bevorzugt sind Zusammensetzungen, bei denen die Polymeren von Aminosäuren, N-C₂-C₂₄-Acylaminosäuren und/oder den physiologisch verträglichen Metallsalzen dieser Substanzen ausgewählt sind aus tierischen und pflanzlichen Proteinhydrolysaten, tierischen und pflanzlichen Proteinen und DNA-Reparaturenzymen. Hier sind ganz besonders bevorzugte erfindungsgemäße Zusammensetzungen **dadurch gekennzeichnet, dass** sie zusätzlich mindestens ein DNA-Reparaturenzym, vorzugsweise Photolyase und/oder T4 Endonuclease V und/oder 8-Oxoguanin-glycosylase und/oder deren Mischungen enthalten.

Die DNA-Reparaturenzyme können sowohl in Liposomen verkapselt als auch frei, das heißt unverkapselt, vorliegen. Die Liposomenverkapselung kann bevorzugt mit Phospholipiden, besonders bevorzugt mit Lecithin, erfolgen. Erfindungsgemäß besonders bevorzugt ist der Einsatz von mindestens einem liposomenverkapselten DNA-Reparaturenzym. Liposomenverkapselte Photolyase ist im Handel z. B. unter der Produktbezeichnung Photosomes™ (INCI-Bezeichnung: Aqua, Lecithin, Plankton Extract) liposomenverkapselte T4N5 z. B. unter der Bezeichnung Ultrasomes™ (INCI-Bezeichnung: Aqua, Lecithin, Micrococcus Lysate) von der Firma AGI Dermatics, USA, erhältlich. Da sich bei längerer Lagerung sowohl des Handelsprodukts als auch der erfindungsgemäßen Zusammensetzungen zwischen der verkapselten wässrigen, das Enzym enthaltenden Phase und der nicht-verkapselten, äußeren wässrigen Phase ein Gleichgewicht einstellt, liegt ein Teil der DNA-Reparaturenzyme unverkapselt vor.

In den erfindungsgemäßen Zusammensetzungen sind die Handelsprodukte Photosomes™ oder Ultrasomes™ bevorzugt in Mengen von 0,1 - 10 Gew.-%, besonders bevorzugt 0,5 - 5,0 Gew.-% und außerordentlich bevorzugt 1,0 - 4,0 Gew.-%, bezogen auf das gesamte Mittel, enthalten. Bevorzugte erfindungsgemäße Zusammensetzungen sind **dadurch gekennzeichnet, dass** mindestens ein DNA-Reparaturenzym in Gesamtmengen von 0,00001 - 1 Gew.-%, bevorzugt 0,0001 - 0,1 Gew.-%, besonders bevorzugt 0,001 - 0,01 - 0,05 Gew.-% und außerordentlich bevorzugt 0,002 - 0,005 Gew.-%, enthalten ist.

Ganz besonders bevorzugte erfindungsgemäße Hautbehandlungsmittel sind dadurch gekennzeichnet, dass sie das/die DNA-Reparaturenzym(e) - bezogen auf das Gewicht des Hautbehandlungsmittels - in Mengen von 0,00001 - 1 Gew.-%, bevorzugt 0,0001 - 0,1 Gew.-%, besonders bevorzugt 0,001 -0,05 Gew.-% und außerordentlich bevorzugt 0,002 - 0,005 Gew.-%, enthalten.

In einer weiteren bevorzugten Ausführungsform enthält das erfindungsgemäße Hautbehandlungsmittel zusätzlich mindestens eine UV-absorbierende Substanz. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate sowie Naturstoffe wie Umbelliferon und die körpereigene Urocansäure geeignet. Besondere Bedeutung haben Biphenyl- und vor allem Stilbenderivate, kommerziell als Tinosorb^{®} FD oder Tinosorb^{®} FR ex Ciba erhältlich. Als UV-B-Absorber sind zu nennen 3-Benzylidencampher bzw. 3-Benzylidennorcampher und dessen Derivate, z.B. 3-(4-Methylbenzyliden)campher; 4-Aminobenzoesäurederivate, vorzugsweise 4-(Di-methylamino)benzoesäure-2-ethylhexylester, 4-Dimethylaminobenzoesäure-2-octylester und 4-(Dimethylamino)benzoesäureamylester; Ester der Zimtsäure, vorzugsweise 4-Meth-oxyzimtsäure-2-ethylhexylester, 4-Methoxyzimtsäurepropylester, 4-Methoxyzimtsäureisoamylester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzyl-ester, Salicylsäurehomomenthylester; Derivate des Benzophenons, vorzugsweise 2-Hy-droxy-4-methoxybenzophenon, 2-Hydroxy-4-methoxy-4`-methylbenzophenon, 2,2'-Dihy-droxy-4-methoxybenzophenon; Ester der Benzalmalonsäure, vorzugsweise 4-Methoxy-benzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate. Weiterhin geeignet sind 2-Phenylbenzimidazol-5-sul-fonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hy-droxy-4-methoxybenzophenon-5-sulfon-säure und ihre Salze; Sulfonsäurederivate des 3-Benzylidencamphers, wie z.B. 4-(2-Oxo-3-bor-nylidenmethyl)benzol-sulfonsäure und 2-Methyl-5-(2-oxo-3-bornyliden)sulfonsäure und deren Salze.

Die mindestens eine UV-absorbierende Substanz ist in bevorzugten erfindungsgemäßen Hautbehandlungsmitteln in einer Gesamtmenge von 0,01 Gew.-% bis 20 Gew.-%, besonders bevorzugt 1 - 10 Gew.-%, außerordentlich bevorzugt 2 Gew.-% bis 7 Gew.-%, enthalten.

Weitere bevorzugte erfindungsgemäße Zusammensetzungen sind **dadurch gekennzeichnet, dass** zur Verbesserung der Hautfeuchtigkeit unter Verzicht auf komedogene Substanzen mindestens ein alkyl- oder hydroxyalkylsubstituierten Harnstoff der allgemeinen Formel (A) enthalten ist, 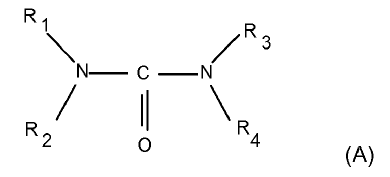 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (A) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-HydroxyalkylGruppe, die mit 1 bis 5 Hydroxylgruppen oder C₁-C₄-Hydroxyalkyl-Gruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt. Weitere bevorzugte erfindungsgemäße

Zusammensetzungen sind **dadurch gekennzeichnet, dass** der mindestens eine alkyl- oder hydroxyalkylsubstituierte Harnstoff der allgemeinen Formel (A) ausgewählt ist aus Bis-N,N'- (2-hydroxyethyl)harnstoff.

Erfindungsgemäß besonders bevorzugte Zusammensetzungen sind **dadurch gekennzeichnet, dass** sie mindestens einen alkyl- oder hydroxyalkylsubstituierten Harnstoff gemäß Formel (A), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)-harnstoff, bei Raumtemperatur ein kristalliner Feststoff, ist beispielsweise als Handelsprodukt Hydrovance von Akzo Nobel als ca. 45 - 55 %ige wässrige Lösung mit einem Gehalt an Harnstoff und Ammoniumlactat erhältlich.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins). Erfindungsgemäß besonders bevorzugte Zusammensetzungen sind **dadurch gekennzeichnet, dass** sie mindestens eine natürliche Betainverbindung in einer Gesamtmenge von 0,05 - 5 Gew.-%, bevorzugt 0,1 - 3 Gew.-%, besonders bevorzugt 0,5 - 2 Gew.-%, jeweils bezogen auf die gesamte Zusammensetzung, enthalten.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind **dadurch gekennzeichnet, dass** sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten. Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Ein weiterer Gegenstand der vorliegenden Erfindung ist die Verwendung von erfindungsgemäßen Zusammensetzungen zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut.

Bezüglich weiterer bevorzugter Ausführungsformen der erfindungsgemäßen Verwendungen gilt mutatis mutandis das zu den erfindungsgemäßen Mitteln Gesagte.

Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, **dadurch gekennzeichnet, dass** ein erfindungsgemäßes Hautbehandlungsmittel auf die Haut aufgetragen wird.

Auch bezüglich weiterer bevorzugter Ausführungsformen der erfindungsgemäßen Verfahren gilt mutatis mutandis das zu den erfindungsgemäßen Mitteln Gesagte.

### Beispiele

Es wurden folgende Hautcremes hergestellt (Angaben in Gew.-%):

| | **1.1** | **1.2** | **1.3** | **1.4** | **1.5** | **1.6** | |
|---|---|---|---|---|---|---|---|
| Distelöl | 3,00 | 3,00 | 3,00 | 3,00 | 3,00 | 3,00 | |
| Myritol 318 | 5,00 | 5,00 | 5,00 | 5,00 | 5,00 | 5,00 | |
| Novata AB | 2,00 | 2,00 | 2,00 | 2,00 | 2,00 | 2,00 | |
| Lanette 22 | 1,00 | 1,00 | 1,00 | 1,00 | 1,00 | 1,00 | |
| Cutina MD | 2,00 | 2,00 | 2,00 | 2,00 | 2,00 | 2,00 | |
| Stenol 1618 | 1,00 | 1,00 | 1,00 | 1,00 | 1,00 | 1,00 | |
| Baysilon M350 | 1,00 | 1,00 | 1,00 | 1,00 | 1,00 | 1,00 | |
| Controx KS | 0,05 | 0,05 | 0,05 | 0,05 | 0,05 | 0,05 | |
| Propylparaben | 0,20 | 0,20 | 0,20 | 0,20 | 0,20 | 0,20 | |
| Dry Flo Plus | 1,00 | 2,00 | 3,00 | 1,00 | 2,00 | 3,00 | |
| Propylenglycol | 5,00 | 5,00 | 5,00 | 5,00 | 5,00 | 5,00 | |
| Glycerine | 5,00 | 3,00 | 3,00 | 5,00 | 3,00 | 3,00 | |
| Methylparaben | 0,20 | 0,20 | 0,20 | 0,20 | 0,20 | 0,20 | |
| Phenoxyethanol | 0,9 | 0,5 | 0,9 | 0,9 | 0,5 | 0,9 | |
| Parfum | 0,10 | 0,10 | 0,10 | 0,10 | 0,10 | 0,10 | |
| Phycojuvenine | 1 | 2 | 1 | 1 | 2 | 1 | |
| Superoxiddismutase | 0,1 | - | - | - | - | - | |
| Lipochroman | - | 0,01 | 0,05 | - | - | - | |
| Lys'Lastine | - | - | - | 1 | 1 | 1 | |
| Aqua | ad 100 | ad 100 | ad 100 | ad 100 | ad 100 | ad 100 | |

| | **1.7** | **1.8** | **1.9** | **1.10** | **1.11** | **1.12** | **1.13** |
|---|---|---|---|---|---|---|---|
| Distelöl | 3,00 | 3,00 | 3,00 | 3,00 | 3,00 | 3,00 | 3,00 |
| Myritol 318 | 5,00 | 5,00 | 5,00 | 5,00 | 5,00 | 5,00 | 5,00 |
| Novata AB | 2,00 | 2,00 | 2,00 | 2,00 | 2,00 | 2,00 | 2,00 |
| Lanette 22 | 1,00 | 1,00 | 1,00 | 1,00 | 1,00 | 1,00 | 1,00 |
| Cutina MD | 2,00 | 2,00 | 2,00 | 2,00 | 2,00 | 2,00 | 2,00 |
| Stenol 1618 | 1,00 | 1,00 | 1,00 | 1,00 | 1,00 | 1,00 | 1,00 |
| Baysilon M350 | 1,00 | 1,00 | 1,00 | 1,00 | 1,00 | 1,00 | 1,00 |
| Controx KS | 0,05 | 0,05 | 0,05 | 0,05 | 0,05 | 0,05 | 0,05 |
| Propylparaben | 0,20 | 0,20 | 0,20 | 0,20 | 0,20 | 0,20 | 0,20 |
| Dry Flo Plus | 2,00 | 3,00 | 1,00 | 2,00 | 3,00 | 2,00 | 3,00 |
| Propylenglycol | 5,00 | 5,00 | 5,00 | 5,00 | 5,00 | 5,00 | 5,00 |
| Glycerine | 3,00 | 3,00 | 5,00 | 3,00 | 3,00 | 3,00 | 3,00 |
| Methylparaben | 0,20 | 0,20 | 0,20 | 0,20 | 0,20 | 0,20 | 0,20 |
| Phenoxyethanol | 0,5 | 0,9 | 0,9 | 0,5 | 0,9 | 0,5 | 0,9 |
| Parfum | 0,10 | 0,10 | 0,10 | 0,10 | 0,10 | 0,10 | 0,10 |
| Phycojuvenine | 1 | 1 | - | - | - | - | -- |
| Superoxiddismutase | - | - | - | - | - | - | - |
| Lipochroman | - | - | - | - | - | - | - |
| Lys'Lastine | 1 | 1 | - | - | - | - | - |
| Matrixyl 3000 | - | - | 3 | - | - | - | - |
| Phytokine | - | - | - | 2 | - | - | 3 |
| Biopeptide CL | - | - | - | - | 3 | - | - |
| Ridulisse C | - | - | - | - | - | 2 | - |
| Aqua | ad 100 | ad 100 | ad 100 | ad 100 | ad 100 | ad 100 | ad 100 |

| | | | | | | | |
|---|---|---|---|---|---|---|---|
| Myritol 318 | | Caprylic/Capric Triglyceride | | | Cognis | | |
| Novata AB | | Cocoglycerides | | | Cognis | | |
| LANETTE® 22 | | Behenyl Alcohol | | | Cognis | | |
| Cutina MD | | Glyceryl Stearate | | | Cognis | | |
| Stenol 16/18 | | Cetearylalkohol | | | Cognis | | |
| Baysilone-Öl M 350 | | Dimethicone | | | GE Bayer Silicones | | |
| Controx KS | | Tocopherol, Hydrogenated Palm Glycerides Citrate | | | Cognis | | |
| Dry Flo Plus | | Aluminium Starch Octenylsuccinate | | | National Starch | | |
| Phycojuvenine | | Laminaria Digitata Extract, ca. 3%ig in Wasser | | | Codif | | |
| Lipochroman | | DIMETHYLMETHOXY CHROMANOL | | | Lipotec | | |
| Lys'Lastine | | Dillextrakt, 0,2-3%ig in Wasser/Butylenglycol; INCI-Bezeichnung: Aqua (Water), Butylene Glycol, Peucedanum Graveolens (Dill) Extract, Xanthan Gum | | | BASF | | |
| Matrixyl 3000 | | Glycerin, Aqua, Butylene Glycol, Carbomer, Polysorbate 20, Palmitoyl Oligopeptide, Palmitoyl Tetrapeptide-1 | | | Sederma | | |
| Phytokine | | HYDROLYZED SOY PROTEIN | | | Coletica | | |
| Ridulisse C | | HYDROLYZED SOY PROTEIN | | | Silab | | |
| Biopeptide CL | | N-Palmitoyl-Gly-His-Lys | | | Sederma | | |

| | **2.1** | **2.2** | **2.3** | **2.4** | **2.5** | **2.6** | |
|---|---|---|---|---|---|---|---|
| Lipoid S75-3 | 0,50 | 0,50 | 0,50 | 0,50 | 0,50 | 0,50 | |
| Tocopherylacetat | 0,50 | 0,50 | 0,50 | 0,50 | 0,50 | 0,50 | |
| Cutina MD | 1,00 | 1,00 | 1,00 | 1,00 | 1,00 | 1,00 | |
| Lanette 22 | 2,00 | 2,00 | 2,00 | 2,00 | 2,00 | 2,00 | |
| Baysilone M 350 | 0,50 | 0,50 | 0,50 | 0,50 | 0,50 | 0,50 | |
| Propylparaben | 0,20 | 0,20 | 0,20 | 0,20 | 0,20 | 0,20 | |
| Dow Corning 9040 | 1,00 | 1,00 | 1,00 | 1,00 | 1,00 | 1,00 | |
| Glycerin | 4,50 | 3,00 | 4,50 | 4,50 | 3,00 | 3,00 | |
| Hexandiol | 6,00 | 3,00 | 6,00 | 6,00 | 3,00 | | |
| Methylparaben | 0,20 | 0,20 | 0,20 | 0,20 | 0,20 | 0,20 | |
| Tego Carbomer 140 | 0,30 | 0,30 | 0,30 | 0,30 | 0,30 | 0,30 | |
| DSH-CN | 5,00 | 5,00 | 5,00 | 5,00 | 5,00 | 5,00 | |
| Propylenglycol | 5,00 | 5,00 | 5,00 | 5,00 | 5,00 | 5,00 | |
| Simulgel NS | 1,50 | 1,50 | 1,50 | 1,50 | 1,50 | 1,50 | |
| TiO2 | 0,50 | 0,50 | 0,50 | 0,50 | 0,50 | 0,50 | |
| Phycojuvenine | 2 | 2 | 1 | 2 | 2 | 1 | |
| Superoxiddismutase | 0,1 | - | - | - | - | - | |
| Lipochroman | - | 0,01 | 0,05 | - | - | - | |
| Lys'Lastine | - | - | - | 1 | 1 | 1 | |
| Wasser | ad 100 | ad 100 | ad 100 | ad 100 | ad 100 | ad 100 | |

| | **2.7** | **2.8** | **2.9** | **2.10** | **2.11** | **2.12** | **2.13** |
|---|---|---|---|---|---|---|---|
| Lipoid S75-3 | 0,50 | 0,50 | 0,50 | 0,50 | 0,50 | 0,50 | 0,50 |
| Tocopherylacetat | 0,50 | 0,50 | 0,50 | 0,50 | 0,50 | 0,50 | 0,50 |
| Cutina MD | 1,00 | 1,00 | 1,00 | 1,00 | 1,00 | 1,00 | 1,00 |
| Lanette 22 | 2,00 | 2,00 | 2,00 | 2,00 | 2,00 | 2,00 | 2,00 |
| Baysilone M 350 | 0,50 | 0,50 | 0,50 | 0,50 | 0,50 | 0,50 | 0,50 |
| Propylparaben | 0,20 | 0,20 | 0,20 | 0,20 | 0,20 | 0,20 | 0,20 |
| Dow Corning 9040 | 1,00 | 1,00 | 1,00 | 1,00 | 1,00 | 1,00 | 1,00 |
| Glycerin | 3,00 | 4,50 | 4,50 | 3,00 | 3,00 | 3,00 | 4,50 |
| Hexandiol | 3,00 | 6,00 | 6,00 | 3,00 | - | 3,00 | 6,00 |
| Methylparaben | 0,20 | 0,20 | 0,20 | 0,20 | 0,20 | 0,20 | 0,20 |
| Tego Carbomer 140 | 0,30 | 0,30 | 0,30 | 0,30 | 0,30 | 0,30 | 0,30 |
| DSH-CN | 5,00 | 5,00 | 5,00 | 5,00 | 5,00 | 5,00 | 5,00 |
| Propylenglycol | 5,00 | 5,00 | 5,00 | 5,00 | 5,00 | 5,00 | 5,00 |
| Simulgel NS | 1,50 | 1,50 | 1,50 | 1,50 | 1,50 | 1,50 | 1,50 |
| TiO2 | 0,50 | 0,50 | 0,50 | 0,50 | 0,50 | 0,50 | 0,50 |
| Phycojuvenine | 1 | 1 | 2 | 2 | 1 | 1 | 1 |
| Lys'Lastine | 1 | 1 | - | - | - | - | - |
| Matrixyl 3000 | - | - | 3 | - | - | - | - |
| Phytokine | - | - | - | 2 | - | - | 3 |
| Biopeptide CL | - | - | - | - | 3 | - | - |
| Ridulisse C | - | - | - | - | - | 2 | - |
| Wasser | ad 100 | ad 100 | ad 100 | ad 100 | ad 100 | ad 100 | ad 100 |

| | | | | | | | |
|---|---|---|---|---|---|---|---|
| Lipoid S 75-3 | Aqua, Lecithine | Lipoid GmbH | | | | | |
| Dow Corning 9040 | Cyclomethicone/ Dimethicone Crosspolymer | Dow Corning | | | | | |
| Tego Carbomer^{®} 140 | Carboxyvinylpolymer (INCI-Bezeichnung: Carbomer) | Goldschmidt | | | | | |
| DSH CN | Water, Dimethylsilanol Hyaluronate | Exsymol | | | | | |
| Simulgel NS | Hydroxyethyl Acrylate / Sodium Acryloyldimethyl Taurate Copolymer / Squalane / Polysorbate 60 | Seppic | | | | | |

| | **3.1** | **3.2** | **3.3** | **3.4** | **3.5** | **3.6** | **3.7** |
|---|---|---|---|---|---|---|---|
| Sisterna SP30-C | 2,0 | 2,0 | 2,0 | 2,0 | 2,0 | 2,0 | 2,0 |
| Sisterna SP70-C | 0,8 | 0,8 | 0,8 | 0,8 | 0,8 | 0,8 | 0,8 |
| Propylparaben | 0,2 | 0,2 | 0,2 | 0,2 | 0,2 | 0,2 | 0,2 |
| Methylparaben | 0,2 | 0,2 | 0,2 | 0,2 | 0,2 | 0,2 | 0,2 |
| Ethanol | 5,0 | 3,0 | 3,0 | 5,0 | 3,0 | - | 5,0 |
| Dry Flo Plus | 1,0 | 1,0 | 1,0 | 1,0 | 1,0 | 1,0 | 1,0 |
| Parfum | 0,3 | 0,3 | 0,3 | 0,3 | 0,3 | 0,3 | 0,3 |
| Cetearyl Alcohol | 1,0 | 1,0 | 1,0 | 1,0 | 1,0 | 1,0 | 1,0 |
| Fucogel 1000 | 2,0 | 1,0 | 1,0 | 2,0 | 1,0 | 0,5 | 2,0 |
| Keltrol SF | 0,2 | - | - | 0,2 | - | 0,2 | 0,2 |
| Aristoflex AVC | - | 0,5 | 0,5 | - | 0,5 | - | - |
| Hexandiol-1,6 | 5,0 | - | - | 5,0 | - | - | 5,0 |
| Glycerin | 10,0 | 5,0 | 5,0 | 10,0 | 5,0 | 10,0 | 10,0 |
| Dow Corning 200 Fluid | 7,0 | - | - | 7,0 | - | - | 7,0 |
| Dow Corning 245 | - | 5,0 | 5,0 | - | 5,0 | 5,0 | - |
| Cetiol 868 | 2,0 | - | - | 2,0 | - | 2,0 | 2,0 |
| Parfum | 0,3 | 0,3 | 0,3 | 0,3 | 0,3 | 0,3 | 0,3 |
| Phycojuvenine | 1 | 2 | 1 | 1 | 2 | 1 | 1 |
| Superoxiddismutase | 0,1 | - | - | - | - | - | - |
| Lipochroman | - | 0,01 | 0,05 | - | - | - | - |
| Lys'Lastine | - | - | - | 1 | 1 | 1 | 1 |
| Wasser | ad 100 | ad 100 | ad 100 | ad 100 | ad 100 | ad 100 | ad 100 |

| | **3.8** | **3.9** | **3.10** | **3.11** | **3.12** | **3.13** |
|---|---|---|---|---|---|---|
| Sisterna SP30-C | 2,0 | 2,0 | 2,0 | 2,0 | 2,0 | 2,0 |
| Sisterna SP70-C | 0,8 | 0,8 | 0,8 | 0,8 | 0,8 | 0,8 |
| Propylparaben | 0,2 | 0,2 | 0,2 | 0,2 | 0,2 | 0,2 |
| Methylparaben | 0,2 | 0,2 | 0,2 | 0,2 | 0,2 | 0,2 |
| Ethanol | 3,0 | 5,0 | 3,0 | - | 5,0 | 3,0 |
| Dry Flo Plus | 1,0 | 1,0 | 1,0 | 1,0 | 1,0 | 1,0 |
| Parfum | 0,3 | 0,3 | 0,3 | 0,3 | 0,3 | 0,3 |
| Cetearyl Alcohol | 1,0 | 1,0 | 1,0 | 1,0 | 1,0 | 1,0 |
| Fucogel 1000 | 1,0 | 2,0 | 1,0 | 0,5 | 2,0 | 1,0 |
| Keltrol SF | -- | 0,2 | - | 0,2 | 0,2 | - |
| Aristoflex AVC | 0,5 | - | 0,5 | - | - | 0,5 |
| Hexandiol-1,6 | - | 5,0 | - | - | 5,0 | - |
| Glycerin | 5,0 | 10,0 | 5,0 | 10,0 | 10,0 | 5,0 |
| Dow Corning 200 Fluid | -- | 7,0 | - | - | 7,0 | - |
| Dow Corning 245 | 5,0 | - | 5,0 | 5,0 | - | 5,0 |
| Cetiol 868 | - | 2,0 | - | 2,0 | 2,0 | - |
| Parfum | 0,3 | 0,3 | 0,3 | 0,3 | 0,3 | 0,3 |
| Phycojuvenine | 1 | 1 | 2 | 1 | 1 | 1 |
| Lys'Lastine | 1 | - | - | - | - | - |
| Matrixyl 3000 | - | 3 | - | - | - | - |
| Phytokine | - | - | 2 | - | - | 3 |
| Biopeptide CL | - | - | - | 3 | - | - |
| Ridulisse C | - | - | - | - | 2 | - |
| Wasser | ad 100 | ad 100 | ad 100 | ad 100 | ad 100 | ad 100 |

- Sisterna SP 30 C: INCI-Bezeichnung: Sucrose Distearate (Sisterna B.V.)
- Sisterna SP 70 C: INCI-Bezeichnung: Sucrose Stearate (Sisterna B.V.)
- Fucogel 1000: INCI-Bezeichnung: Biosaccharide Gum-1 (Solabia)
- Aristoflex^{®} AVC: Ammoniumacryloyldimethyltaurat/Vinylpyrrolidon-Copolymer (ca. 92% Festsubstanz in tert.-Butanol; INCI-Bezeichnung : Ammonium Acryloyldimethyltaurate/VP Copolymer, t-Butyl Alcohol) (Clariant)
- Dow Corning^{®} 200 Fluid: Polydimethylsiloxan (INCI-Bezeichnung: Dimethicone) (Dow Corning)
- Dow Corning^{®} 245 Fluid: Dimethylsiloxanpentamer (95% Reinheit; INCI-Bezeichnung: Cyclomethicone) (Dow Corning)
- Cetiol^{®} 868: Ethylhexylstearat (INCI-Bezeichnung: Ethyl Hexyl Stearate) (Cognis)

| | **4.1** | **4.2** | **4.3** | **4.4** | **4.5** | **4.6** | **4.7** |
|---|---|---|---|---|---|---|---|
| Dow Corning 245 Fluid | 25 | 25 | 25 | 25 | 25 | 25 | 25 |
| Abil EM 90 | 2 | 2 | 2 | 2 | 2 | 2 | 2 |
| Belsil DM 100 | 2,5 | 2,5 | 2,5 | 2,5 | 2,5 | 2,5 | 2,5 |
| Hydrogenated Castor Oil | 2 | 2,5 | 2,5 | 2 | 2,5 | 2 | 2 |
| Glycerin | 5 | 5 | 5 | 5 | 5 | 5 | 5 |
| NaCl | 2 | 2 | 2 | 2 | 2 | 2 | 2 |
| Symdiol 68 | 0,5 | 0,5 | 0,5 | 0,5 | 0,5 | 0,5 | 0,5 |
| Phenoxyethanol | 0,4 | 0,4 | 0,4 | 0,4 | 0,4 | 0,4 | 0,4 |
| Parfum | 0,3 | 0,3 | 0,3 | 0,3 | 0,3 | 0,3 | 0,3 |
| Phycojuvenine | 1 | 2 | 1 | 1 | 2 | 1 | 1 |
| Superoxiddismutase | 0,1 | - | - | - | - | - | - |
| Lipochroman | - | 0,01 | 0,05 | - | - | - | - |
| Lys'Lastine | - | - | - | 1 | 1 | 1 | 1 |
| Wasser | ad 100 | ad 100 | ad 100 | ad 100 | ad 100 | ad 100 | ad 100 |

| | **4.8** | **4.9** | **4.10** | **4.11** | **4.12** | **4.13** |
|---|---|---|---|---|---|---|
| Dow Corning 245 Fluid | 25 | 25 | 25 | 25 | 25 | 25 |
| Abil EM 90 | 2 | 2 | 2 | 2 | 2 | 2 |
| Belsil DM 100 | 2,5 | 2,5 | 2,5 | 2,5 | 2,5 | 2,5 |
| Hydrogenated Castor Oil | 2,5 | 2 | 2,5 | 2 | 2 | 2,5 |
| Glycerin | 5 | 5 | 5 | 5 | 5 | 5 |
| NaCl | 2 | 2 | 2 | 2 | 2 | 2 |
| Symdiol68 | 0,5 | 0,5 | 0,5 | 0,5 | 0,5 | 0,5 |
| Phenoxyethanol | 0,4 | 0,4 | 0,4 | 0,4 | 0,4 | 0,4 |
| Parfum | 0,3 | 0,3 | 0,3 | 0,3 | 0,3 | 0,3 |
| Phycojuvenine | 1 | 1 | 2 | 1 | 1 | 1 |
| Lys'Lastine | 1 | - | - | - | - | - |
| Matrixyl 3000 | - | 3 | - | - | - | - |
| Phytokine | - | - | 2 | - | - | 3 |
| Biopeptide CL | - | - | - | 3 | - | - |
| Ridulisse C | - | - | - | - | 2 | - |
| Wasser | ad 100 | ad 100 | ad 100 | ad 100 | ad 100 | ad 100 |

- Abil^{®} EM 90: modifiziertes Polyether-Polysiloxan (INCI-Bezeichnung: Cetyl PEG/PPG-10/1 Dimethicone) (Evonik Degussa)
- Belsil^{®} DM 100: Polydimethylsiloxan (INCI-Bezeichnung: Dimethicone) (Wacker)
- Symdiol 68: 1,2-Octandiol, 1,2-Hexandiol (Symrise)

| | **5.1** | **5.2** | **5.3** | **5.4** | **5.5** | **5.6** | **5.7** |
|---|---|---|---|---|---|---|---|
| Montanov 202 | 5,0 | 5,0 | 5,0 | 5,0 | 5,0 | 5,0 | 5,0 |
| Cutina MD | 2,0 | 2,0 | 2,0 | 2,0 | 2,0 | 2,0 | 2,0 |
| Cetearyl Alcohol | 1,0 | 1,0 | 1,0 | 1,0 | 1,0 | 1,0 | 1,0 |
| Cetiol B | 9,0 | 9,0 | 9,0 | 9,0 | 9,0 | 9,0 | 9,0 |
| Controx KS | 0,05 | 0,05 | 0,05 | 0,05 | 0,05 | 0,05 | 0,05 |
| Dow Corning 245 | - | 7,0 | 7,0 | - | 7,0 | 3,0 | - |
| Dow Corning 9040 | 1,0 | 1,0 | 1,0 | 1,0 | 1,0 | - | 1,0 |
| Parfum | 0,3 | 0,3 | 0,3 | 0,3 | 0,3 | 0,3 | 0,3 |
| Fucogel 1000 | - | 2,0 | 2,0 | - | 2,0 | - | - |
| Keltrol SF | - | 0,2 | 0,2 | - | 0,2 | 0,2 | - |
| Aristoflex AVC | - | - | - | - | - | 0,5 | - |
| Hexandiol-1,6 | 6,0 | 6,0 | 6,0 | 6,0 | 6,0 | - | 6,0 |
| Glycerin | 3,0 | 5,0 | 5,0 | 3,0 | 5,0 | 10,0 | 3,0 |
| Dow Corning 200 Fluid | 7,0 | 7,0 | 7,0 | 7,0 | 7,0 | - | 7,0 |
| Dow Corning 245 | - | 5,0 | 5,0 | - | 5,0 | 3,0 | - |
| Dow Corning 9040 | 1,0 | - | - | 1,0 | - | 1,0 | 1,0 |
| Propylenglycol | 2,0 | 2,0 | 2,0 | 2,0 | 2,0 | - | 2,0 |
| Tego Carbomer | 0,3 | 0,3 | 0,3 | 0,3 | 0,3 | 0,3 | 0,3 |
| Dry Flo Plus | 1,0 | 1,0 | 1,0 | 1,0 | 1,0 | 1,0 | 1,0 |
| Phycojuvenine | 1 | 1 | 1 | 1 | 2 | 1 | 1 |
| Superoxiddismutase | 0,1 | - | - | - | - | - | - |
| Lipochroman | - | 0,01 | 0,05 | - | - | - | - |
| Lys'Lastine | - | - | - | 1 | 1 | 1 | 1 |
| Wasser | ad 100 | ad 100 | ad 100 | ad 100 | ad 100 | ad 100 | ad 100 |

| | **5.8** | **5.9** | **5.10** | **5.11** | **5.12** | **5.13** |
|---|---|---|---|---|---|---|
| Montanov 202 | 5,0 | 5,0 | 5,0 | 5,0 | 5,0 | 5,0 |
| Cutina MD | 2,0 | 2,0 | 2,0 | 2,0 | 2,0 | 2,0 |
| Cetearyl Alcohol | 1,0 | 1,0 | 1,0 | 1,0 | 1,0 | 1,0 |
| Cetiol B | 9,0 | 9,0 | 9,0 | 9,0 | 9,0 | 9,0 |
| Controx KS | 0,05 | 0,05 | 0,05 | 0,05 | 0,05 | 0,05 |
| Dow Corning 245 | 7,0 | - | 7,0 | 3,0 | - | 7,0 |
| Dow Corning 9040 | 1,0 | 1,0 | 1,0 | - | 1,0 | 1,0 |
| Parfum | 0,3 | 0,3 | 0,3 | 0,3 | 0,3 | 0,3 |
| Fucogel 1000 | 2,0 | - | 2,0 | - | - | 2,0 |
| Keltrol SF | 0,2 | - | 0,2 | 0,2 | - | 0,2 |
| Aristoflex AVC | - | - | - | 0,5 | - | - |
| Hexandiol-1,6 | 6,0 | 6,0 | 6,0 | - | 6,0 | 6,0 |
| Glycerin | 5,0 | 3,0 | 5,0 | 10,0 | 3,0 | 5,0 |
| Dow Corning 200 Fluid | 7,0 | 7,0 | 7,0 | - | 7,0 | 7,0 |
| Dow Corning 245 | 5,0 | - | 5,0 | 3,0 | - | 5,0 |
| Dow Corning 9040 | - | 1,0 | - | 1,0 | 1,0 | - |
| Propylenglycol | 2,0 | 2,0 | 2,0 | - | 2,0 | 2,0 |
| Tego Carbomer | 0,3 | 0,3 | 0,3 | 0,3 | 0,3 | 0,3 |
| Dry Flo Plus | 1,0 | 1,0 | 1,0 | 1,0 | 1,0 | 1,0 |
| Phycojuvenine | 1 | 1 | 2 | 1 | 1 | 1 |
| Lys'Lastine | 1 | - | - | - | - | - |
| Matrixyl 3000 | - | 3 | - | - | - | - |
| Phytokine | - | - | 2 | - | - | 3 |
| Biopeptide CL | - | - | - | 3 | - | - |
| Ridulisse C | - | - | - | - | 2 | - |
| Wasser | ad 100 | ad 100 | ad 100 | ad 100 | ad 100 | ad 100 |

Montanov^{®} 202: INCI: Arachidyl Alcohol, Behenyl Alcohol, Arachidyl Glucoside (Seppic)

| | **6.1** | **6.2** | **6.3** | **6.4** | **6.5** | **6.6** | **6.7** |
|---|---|---|---|---|---|---|---|
| Glucamate SSE-20 | 2,0 | 2,0 | 2,0 | 2,0 | 2,0 | 2,0 | 2,0 |
| Sympatens-BS/080 | 2,0 | 2,0 | 2,0 | 2,0 | 2,0 | 2,0 | 2,0 |
| Dry Flo Plus | - | 1,0 | 1,0 | - | 1,0 | 1,0 | - |
| Parfum | 0,3 | 0,3 | 0,3 | 0,3 | 0,3 | 0,3 | 0,3 |
| Silikonöl 350 | 0,3 | 0,3 | - | 0,3 | 0,3 | - | 0,3 |
| Cutina MD | 2,0 | 2,0 | 2,0 | 2,0 | 2,0 | 2,0 | 2,0 |
| Paraffinöl | 2,0 | 1,0 | - | 2,0 | 1,0 | - | 2,0 |
| Cetearyl Alcohol | 1,0 | 1,5 | 1,25 | 1,0 | 1,5 | 1,25 | 1,0 |
| Capryl-/Caprinsäure-Triglycerid | - | 3,5 | 3,0 | - | 3,5 | 3,0 | - |
| Phenoxyethanol | 0,98 | 0,5 | 0,9 | 0,98 | 0,5 | 0,9 | 0,98 |
| Keltrol SF | - | - | 0,2 | - | - | 0,2 | - |
| Aristoflex AVC | - | 0,5 | 0,5 | - | 0,5 | 0,5 | - |
| Hexandiol-1,6 | - | 5,0 | 5,0 | - | 5,0 | 5,0 | - |
| Glycerin | 2,0 | 5,0 | 10,0 | 2,0 | 5,0 | 10,0 | 2,0 |
| Culminal MHPC 100 | 0,1 | 0,1 | 0,1 | 0,1 | 0,1 | 0,1 | 0,1 |
| Propylenglycol | 2,0 | 5,0 | 3,0 | 2,0 | 5,0 | 3,0 | 2,0 |
| Phycojuvenine | 1 | 2 | 1 | 1 | 2 | 1 | 1 |
| Superoxiddismutase | 0,1 | - | - | - | - | - | - |
| Lipochroman | - | 0,01 | 0,05 | - | - | - | - |
| Lys'Lastine | - | - | - | 1 | 1 | 1 | 1 |
| Wasser | ad 100 | ad 100 | ad 100 | ad 100 | ad 100 | ad 100 | ad 100 |

| | **6.8** | **6.9** | **6.10** | **6.11** | **6.12** | **6.13** |
|---|---|---|---|---|---|---|
| Glucamate SSE-20 | 2,0 | 2,0 | 2,0 | 2,0 | 2,0 | 2,0 |
| Sympatens-BS/080 | 2,0 | 2,0 | 2,0 | 2,0 | 2,0 | 2,0 |
| Dry Flo Plus | 1,0 | - | 1,0 | 1,0 | - | 1,0 |
| Parfum | 0,3 | 0,3 | 0,3 | 0,3 | 0,3 | 0,3 |
| Silikonöl 350 | - | 0,3 | 0,3 | - | 0,3 | - |
| Cutina MD | 2,0 | 2,0 | 2,0 | 2,0 | 2,0 | 2,0 |
| Paraffinöl | - | 2,0 | 1,0 | - | 2,0 | - |
| Cetearyl Alcohol | 1,25 | 1,0 | 1,5 | 1,25 | 1,0 | 1,25 |
| Capryl-/Caprinsäure-Triglycerid | 3,0 | - | 3,5 | 3,0 | - | 3,0 |
| Phenoxyethanol | 0,9 | 0,98 | 0,5 | 0,9 | 0,98 | 0,9 |
| Keltrol SF | 0,2 | - | - | 0,2 | - | 0,2 |
| Aristoflex AVC | 0,5 | - | 0,5 | 0,5 | - | 0,5 |
| Hexandiol-1,6 | 5,0 | - | 5,0 | 5,0 | - | 5,0 |
| Glycerin | 10,0 | 2,0 | 5,0 | 10,0 | 2,0 | 10,0 |
| Culminal MHPC 100 | 0,1 | 0,1 | 0,1 | 0,1 | 0,1 | 0,1 |
| Propylenglycol | 3,0 | 2,0 | 5,0 | 3,0 | 2,0 | 3,0 |
| Phycojuvenine | 1 | 1 | 2 | 1 | 1 | 1 |
| Superoxiddismutase | | | | | | |
| Lipochroman | | | | | | |
| Lys'Lastine | 1 | | | | | |
| Matrixyl 3000 | | 3 | | | | |
| Phytokine | | | 2 | | | 3 |
| Biopeptide CL | | | | 3 | | |
| Ridulisse C | | | | | 2 | |
| Wasser | ad 100 | ad 100 | ad 100 | ad 100 | ad 100 | ad 100 |

- Glucamate SSE-20: Poly(oxy-1,2-ethanediyl), alpha-hydro-omega-hydroxyether mit Methyl D-glucopyranosid (4:1), octadecanoate (2:3), INCI-Bezeichnung PEG-20 METHYL GLUCOSE SESQUISTEARATE (Noveon)
- Sympatens-BS/080: INCI-Bezeichnung PEG-8 STEARATE (Dr. Kolb)
- Keltrol SF: Xanthan Gum (Kelco)
- Culminal MHPC 100: INCI-Bezeichnung HYDROXYPROPYL METHYLCELLULOSE (Hercules)

| | **7.1** | **7.2** | **7.3** | **7.4** | **7.5** | **7.6** | **7.7** |
|---|---|---|---|---|---|---|---|
| Emulsiphos 677660 | 3 | 3 | 3 | 3 | 3 | 3 | 3 |
| Cetiol B | 5 | 5 | 5 | 5 | 5 | 5 | 5 |
| Estasan GT8-60 3575 MCT Oil | 3,5 | 3,5 | 3,5 | 3,5 | 3,5 | 3,5 | 3,5 |
| Safloröl raffiniert | 2 | 2 | 2 | 2 | 2 | 2 | 2 |
| Behenylalkohol | 3 | 3 | 3 | 3 | 3 | 3 | 3 |
| Cetiol Sensoft | 1 | 1 | 1 | 1 | 1 | 1 | 1 |
| Vitamin E Acetat | 0,5 | 0,5 | 0,5 | 0,5 | 0,5 | 0,5 | 0,5 |
| Controx KS | 0,05 | 0,05 | 0,05 | 0,05 | 0,05 | 0,05 | 0,05 |
| Bienenwachs | 0,2 | 0,2 | 0,2 | 0,2 | 0,2 | 0,2 | 0,2 |
| COSMEDIA SP | 0,5 | 0,5 | 0,5 | 0,5 | 0,5 | 0,5 | 0,5 |
| Butylmethoxydibenzoylmethan | 3 | 3 | 3 | 3 | 3 | 3 | 3 |
| Parsol SLX | 3 | 3 | 3 | 3 | 3 | 3 | 3 |
| Propylparaben | 0,2 | 0,2 | 0,2 | 0,2 | 0,2 | 0,2 | 0,2 |
| Hexandiol-1,6 | 5 | 5 | 5 | 5 | 5 | 5 | 5 |
| Glycerin 86% pflanzlich | 4,5 | 4,5 | 4,5 | 4,5 | 4,5 | 4,5 | 4,5 |
| Sorbit 70% LS DAB | 2 | 2 | 2 | 2 | 2 | 2 | 2 |
| Methylparaben | 0,2 | 0,2 | 0,2 | 0,2 | 0,2 | 0,2 | 0,2 |
| Tego Carbomer 140 | 0,5 | 0,5 | 0,5 | 0,5 | 0,5 | 0,5 | 0,5 |
| NTA-Na3 flüssig 40% | 0,1 | 0,1 | 0,1 | 0,1 | 0,1 | 0,1 | 0,1 |
| Cosmedia Silc | 2 | 2 | 2 | 2 | 2 | 2 | 2 |
| AMP Ultra PC 1000 | 0,99 | 0,99 | 0,99 | 0,99 | 0,99 | 0,99 | 0,99 |
| Neo Heliopan Hydro | 3 | 3 | 3 | 3 | 3 | 3 | 3 |
| Natriumhydroxid Perlen | 0,365 | 0,365 | 0,365 | 0,365 | 0,365 | 0,365 | 0,365 |
| Phycojuvenine | 1 | 2 | 1 | 1 | 2 | 1 | 1 |
| Superoxiddismutase | 0,1 | - | - | - | - | - | - |
| Lipochroman | - | 0,01 | 0,05 | - | - | - | - |
| Lys'Lastine | - | - | - | 1 | 1 | 1 | 1 |
| Wasser, vollentsalzt | ad 100 | ad 100 | ad 100 | ad 100 | ad 100 | ad 100 | ad 100 |

| | **7.8** | **7.9** | **7.10** | **7.11** | **7.12** | **7.13** |
|---|---|---|---|---|---|---|
| Emulsiphos 677660 | 3 | 3 | 3 | 3 | 3 | 3 |
| Cetiol B | 5 | 5 | 5 | 5 | 5 | 5 |
| Estasan GT8-60 3575 MCT Oil | 3,5 | 3,5 | 3,5 | 3,5 | 3,5 | 3,5 |
| Safloröl raffiniert | 2 | 2 | 2 | 2 | 2 | 2 |
| Behenylalkohol | 3 | 3 | 3 | 3 | 3 | 3 |
| Cetiol Sensoft | 1 | 1 | 1 | 1 | 1 | 1 |
| Vitamin E Acetat | 0,5 | 0,5 | 0,5 | 0,5 | 0,5 | 0,5 |
| Controx KS | 0,05 | 0,05 | 0,05 | 0,05 | 0,05 | 0,05 |
| Bienenwachs | 0,2 | 0,2 | 0,2 | 0,2 | 0,2 | 0,2 |
| COSMEDIA SP | 0,5 | 0,5 | 0,5 | 0,5 | 0,5 | 0,5 |
| Butylmethoxydibenzoylmethan | 3 | 3 | 3 | 3 | 3 | 3 |
| Parsol SLX | 3 | 3 | 3 | 3 | 3 | 3 |
| Propylparaben | 0,2 | 0,2 | 0,2 | 0,2 | 0,2 | 0,2 |
| Hexandiol-1,6 | 5 | 5 | 5 | 5 | 5 | 5 |
| Glycerin 86% pflanzlich | 4,5 | 4,5 | 4,5 | 4,5 | 4,5 | 4,5 |
| Sorbit 70% LS DAB | 2 | 2 | 2 | 2 | 2 | 2 |
| Methylparaben | 0,2 | 0,2 | 0,2 | 0,2 | 0,2 | 0,2 |
| Tego Carbomer 140 | 0,5 | 0,5 | 0,5 | 0,5 | 0,5 | 0,5 |
| NTA-Na3 flüssig 40% | 0,1 | 0,1 | 0,1 | 0,1 | 0,1 | 0,1 |
| Cosmedia Silc | 2 | 2 | 2 | 2 | 2 | 2 |
| AMP Ultra PC 1000 | 0,99 | 0,99 | 0,99 | 0,99 | 0,99 | 0,99 |
| Neo Heliopan Hydro | 3 | 3 | 3 | 3 | 3 | 3 |
| Natriumhydroxid Perlen | 0,365 | 0,365 | 0,365 | 0,365 | 0,365 | 0,365 |
| Phycojuvenine | 1 | 1 | 2 | 1 | 1 | 1 |
| Lys'Lastine | 1 | - | - | - | - | - |
| Matrixyl 3000 | - | 3 | - | - | - | - |
| Phytokine | - | - | 2 | - | - | 3 |
| Biopeptide CL | - | - | - | 3 | - | - |
| Ridulisse C | - | - | - | - | 2 | - |
| Wasser, vollentsalzt | ad 100 | ad 100 | ad 100 | ad 100 | ad 100 | ad 100 |

- Emulsiphos 677660: Potassium Cetyl Phosphate, Hydrogenated Palm Glycerides (Symrise)
- Cetiol^{®} B: Dibutyladipat (Cognis)
- Estasan GT8-60 3575: Caprylic acid, capric acid triglyceride (Uniqema)
- Cetiol Sensoft: Propylheptyl Caprylate (Cognis)
- Cosmedia SP: Natriumpolyacrylat (Cognis)
- Parsol^{®} SLX: Dimethicodiethylbenzal malonat (INCI-Bezeichnung: Polysilicone-15, CAS- Nr. 207574-74-1) (DSM Nutritional Products)
- Cosmedia Silc: Silica (Cognis)
- AMP Ultra PC 2000: 2-Amino-2-methyl-propanol (ca. 94,5-95,4% Aktivsubstanz in Wasser; INCI-Bezeichnung: Aminomethyl Propanol) (Dow Chemical)
- Neo Heliopan^{®} Hydro: 2-Phenylbenzimidazol-2-sulfonsäure (INCI: Phenylbenzimidazole Sulfonic Acid) (Symrise)

## Patentansprüche

1. Kosmetische oder dermatologische topische Zusammensetzungen, enthaltend in einem geeigneten kosmetischen oder dermatologischen Träger - jeweils bezogen auf das Gewicht der gesamten Zusammensetzung -
a) 0,001 bis 10 Gew.-% mindestens eines Extraktes aus Laminaria Digitata und
b) 0,001 bis 10 Gew.-% mindestens eines Wirkstoffes aus der Gruppe
- der 6,7-disubstituierten 2,2-Dialkylchromane oder -chromene der allgemeinen Formeln
(I) oder (II), 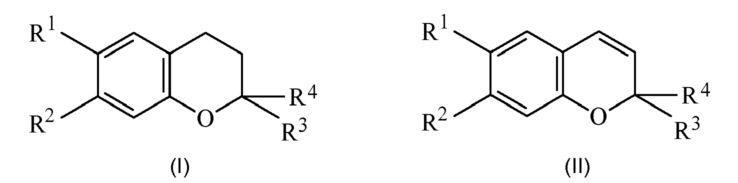 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6, und/oder
- Ellagsäure und/oder Ellagate und/oder
- der Xanthophylle, insbesondere Astaxanthin, und/oder
- der Superoxiddismutasen und/oder
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten und/oder
- Monomeren, Oligomeren und Polymeren von Aminosäuren, N-C₂-C₂₄-Acylaminosäuren, den Estern und/oder den physiologisch verträglichen Metallsalzen dieser Substanzen.

2. Zusammensetzung gemäß Anspruch 1, **dadurch gekennzeichnet, dass** sie 0,00005 bis 0,5 Gew.-%, vorzugsweise 0,0005 bis 0,25 Gew.-% und insbesondere 0,005 bis 0,1 Gew.-% Laminarin der Formel (III) enthält 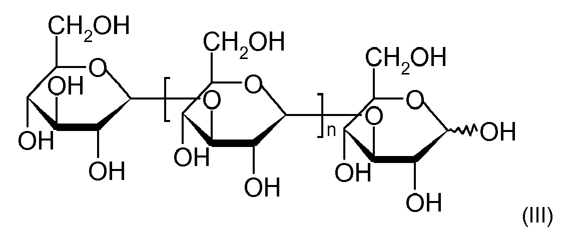 in der n für Werte zwischen 5 und 50, vorzugsweise zwischen 10 und 40 und insbesondere zwischen 15 und 35 steht, wobei die Werte 20, 21, 22, 23, 24, 25, 26, 27, 28, 29 und 30 besonders bevorzugt sind.

3. Zusammensetzung nach einem der Ansprüche 1 oder 2, **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (I) oder (II) wie vorstehend definiert, enthält, wobei bevorzugte Zusammensetzungen - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten.

4. Zusammensetzung nach einem der Ansprüche 1 bis 3, **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Ellagsäure (2,3,7,8-Tetrahydroxy[1]benzopyrano-[5,4,3-*cde*][1]benzopyran-5,10-dion) und/oder Ellagate enthält.

5. Zusammensetzung nach einem der Ansprüche 1 bis 4, **dadurch gekennzeichnet, dass** sie - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 1 Gew.-% Xanthophylle, vorzugsweise Canthaxanthin, Citranaxanthin, Flavoxanthin, Rhodoxanthin, Rubixanthin, Zeaxanthin und ganz besonders bevorzugt Astaxanthin, enthält.

6. Zusammensetzung nach einem der Ansprüche 1 bis 5, **dadurch gekennzeichnet, dass** sie - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 2 Gew.-% Superoxiddismutase(n) enthält.

7. Zusammensetzung nach einem der Ansprüche 1 bis 6, **dadurch gekennzeichnet, dass** die Monomeren von Aminosäuren, N-C₂-C₂₄-Acylaminosäuren und/oder den physiologisch verträglichen Metallsalzen dieser Substanzen ausgewählt sind aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat.

8. Zusammensetzung nach einem der Ansprüche 1 bis 7, **dadurch gekennzeichnet, dass** die Oligomeren von Aminosäuren, N-C₂-C₂₄-Acylaminosäuren und/oder den physiologisch verträglichen Metallsalzen dieser Substanzen ausgewählt sind aus Di-, Tri-, Tetra-, Penta-, Hexa- oder Pentadecapeptiden, die N-acyliert und/oder verestert sein können.

9. Zusammensetzung nach Anspruch 8, **dadurch gekennzeichnet, dass** das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg.

10. Zusammensetzung nach einem der Ansprüche 1 bis 9, **dadurch gekennzeichnet, dass** die Polymeren von Aminosäuren, N-C₂-C₂₄-Acylaminosäuren und/oder den physiologisch verträglichen Metallsalzen dieser Substanzen ausgewählt sind aus
- tierischen und pflanzlichen Proteinhydrolysaten,
- tierischen und pflanzlichen Proteinen und
- DNA-Reparaturenzymen.

11. Zusammensetzung nach einem der Ansprüche 1 bis 10, **dadurch gekennzeichnet, dass** sie zusätzlich mindestens ein DNA-Reparaturenzym, vorzugsweise Photolyase und/oder T4 Endonuclease V und/oder 8-Oxoguanin-glycosylase und/oder deren Mischungen enthält.

12. Verwendung einer Zusammensetzung nach einem der Ansprüche 1 bis 11 zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut.

13. Nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, **dadurch gekennzeichnet, dass** ein Hautbehandlungsmittel gemäß einem der Ansprüche 1 bis 11 auf die Haut aufgetragen wird.
